# EUROPEAN PATENT APPLICATION

(11) **EP 1 867 318 A1**
(43) Date of publication of application: **19.12.2007**
(21) Application number: 06012137.3
(22) Date of filing: 13.06.2006
(51) Int. Cl.: A61K 8/04, A61Q 19/10, A61Q 5/02, A61Q 1/00

(54) **Body Care composition with water-soluble abrasive particles**

(71) Applicant: Wella Aktiengesellschaft, 64274 Darmstadt (DE)
(72) Inventor: Kripp, Thomas, Dr., 64407 Fränkisch-Crumbach (DE); Grasser, Beate, 65795 Hattersheim (DE)

(57) **Abstract**

The invention relates to a body care composition, in particular a cosmetic body care composition, more particularly a body cleaning material, comprising solid particles, which are moderately soluble in water. A most preferred embodiment of the present invention is a peeling shampoo.

## Description

The invention relates to a body care composition, in particular a cosmetic body care composition, more particularly a body cleaning material, comprising solid particles, which are moderately soluble in water. A most preferred embodiment of the present invention is a peeling shampoo.

There exists a wide selection of materials that are used for cleaning surfaces. Depending on the soil, the grade of the staining, the temperature that can be applied in order to clean the soiled object, the residence time of the cleaning material etc., different types of compositions are used as cleaning materials. Most of these compositions comprise surfactants as one of the main integral part. In addition, other substances supporting the cleaning process can be present, such as enzymes or builders.

Cleaning materials for soil, which is difficult to remove, for example tea or coffee stains on teeth, or oil, tar and soot stains on human skin etc., often comprise insoluble abrasives. These abrasives support the cleaning process by an enhanced mechanical scrubbing process during their application.

Hard abrasive materials, like aluminium oxide particles as well as particles made of materials that are softer and therefore more suitable for gentle cleaning processes, like silica or plastics, are well known as ingredients of cleaning materials.

But those solid particles are causing some problems when used in cleaning materials: Due to their insolubility the removal of the particles themselves is often difficult, in particular when a surface is cleaned that has a complex structure, such as for example small furrows or haired skin. Further, even if the particles are removed, they can form solid agglomerates in the waste solution and block the pipes of the drainage system.

As a solution to those problems, cleaning materials have been developed, that comprise water-soluble abrasives, which dissolve after the cleaning process in the aqueous solvent, which is used to rinse the cleaned surface.

JP 2004 083569 A, for example, discloses a cleaning agent for use on stains of mineral oil, animal and vegetable oils, paint and the like using salts as abrasives, which are present in the cleaning agent in an amount above their solubility, but which are highly soluble in water.

The subject of US 5,863,883 A is a bath of cleaning liquid for cleaning solid surfaces, wherein abrasive particles, again easily dissolvable in water, are dispersed in an amount to inhibit the dissolution of them.

EP 193 375 A2 discloses pourable, homogenous, abrasive aqueous detergent compositions also comprising water-soluble abrasives, which are present in an amount above their solubility. These compositions are used for the cleaning of hard surfaces.

A particular application of this cleaning strategy using abrasives is the application for the removal of uneven skin parts from an intact skin surface, for example dandruff. Such loose skin parts are leading to a non-aesthetic over-all impression and can be removed using peeling products comprising abrasives.

The use of insoluble abrasives is again accompanied with some disadvantages going along with their removal from the skin after the cleaning process, especially when skin areas with hair growth are concerned. Those particles can adhere at the skin and the hair and lead to itching or complicate combing.

Therefore, also in the field of body care products, cleaning materials comprising water-soluble abrasives are desired.

JP 03 093 707 A describes oil-in-water type emulsified cosmetic composition with scrubbing effect comprising sodium chloride and/or potassium chloride in amounts exceeding the solubility thereof. Both salts are, however, highly water soluble with the consequence that a scrubbing effect remains only over a short period of time.

An oral cavity composition comprising non-dissolved sodium sulphate is disclosed in JP 08 245 345 A.

It is the object of the present invention to overcome the drawbacks as discussed above and to provide a body care composition comprising an only moderately water-soluble compound in order to keep its abrasive function even when additional water is added to the material.

On the other hand, the water-solubility of the compound must not be too low to make sure the abrasive is really dissolved or nearly dissolved when the cleaning process is finished.

This object is solved by a body care composition comprising at least one water-soluble compound, which has a water-solubility at 20 °C of greater than or equal to 0.1 % and smaller than or equal to 7 % and is at least partially non-dissolved within the body care composition.

The expression "%" as used herein refers to weight percent..

The body care composition can be in powdery form not comprising any solvent at all. In this case, the solvent is added prior to use.

According to a further embodiment, the body care composition already comprises a solvent, preferably water and/or one or more organic solvents. In this case, it is necessary to ensure, that the water-soluble compound is still at least partially non-dissolved. A preferred embodiment according to the invention refers to a peeling shampoo composition.

It is important, that the content of the above defined water-soluble compound is high enough to ensure that even at elevated temperatures above 20 °C, in particular up to a temperature of 40 °C, preferably up to 50 °C, and more preferably up to 60 °C there is still a certain amount of the water-soluble compound non-dissolved in the body care composition.

The content of the above defined water-soluble compound is preferably greater than or equal to 0.5 % and smaller than or equal to 70 %, more preferably greater than or equal to 5 % and smaller than or equal to 60 %.

In a particular embodiment, the above defined water-soluble compound has a lower water-solubility at 20 °C and neutral pH of the water of greater than or equal to 0.2 %, preferably greater than or equal to 0.3%, more preferably greater than or equal to 0.4 % and most preferably greater than or equal to 0.5%. In combination with these lower limits the upper water-solubility at 20 °C and neutral pH of the water can be smaller than or equal to 5 %, preferably smaller than or equal to 4.5 %, more preferably smaller than or equal to 4.2 % and most preferably smaller than or equal to 4 %. The skilled person will consider acceptable combinations of these indicated lower and upper limits to be practicable.

The above mentioned water-soluble compound can be selected from the group consisting of organic acids, inorganic acids, inorganic salts, organic salts, carbon hydrate derivatives, polyols, amino acids, acid amides, acid imides, salts of sulphonic acids and/or heterocycles.
As organic acids adipic acid, azelaic acid, benzoic acid, sorbic acid, succinic acid, fumaric acid, mucic acid, phthalic acid, sulphanilic acid, *p*-aminobenzoic acid, hippuric acid and orotic acid are particularly suitable.

Useful as an inorganic acid is for example boric acid.

Organic Salts, that can be used, are amongst other, magnesium lactate, calcium lactate pentahydrate, calcium glycerophosphate, tricalcium citrate, zinc benzoate, zinc lactate and potassium hydrogentartrate

Lithium carbonate and potassium perchlorate are examples for inorganic salts that can be used for the invention.

Suitable carbon hydrate derivatives are dulcitol, calcium gluconate and pentaerythrol.

Amino acids, that satisfy the requirements of the water-soluble compound according to the present invention are, for example, glutamine, glutamic acid, asparagine, aspartic acid, histidine, leucine, isoleucine, phenylalanine, tryptophane and methionine.

Saccharine as an acid imide and caffeine, theophylline and maltol as heterocycles are also suitable for use as abrasives in body care compositions.

In a further embodiment the water-solubility of the at least one water-soluble compound is dependent on the pH value of the medium.

Especially, the upper water-solubility of the above defined water-soluble compound is achieved at a pH of greater than or equal to 6.5 and smaller than or equal to 9.5. This pH range is the usual pH range of normal tap water. The body care composition itself has preferably a pH value that is outside of this range, preferably towards a minimum solubility of the water-soluble compound. This ensures that during the application at pH values outside this range, there is enough of the solid compound present, even if the total amount is relatively low, and at the end of the application of the body care composition, when rinsing with water of the above mentioned pH range, the solubility increases and said compound can be easily removed.

For this effect particularly amino acids like aspartic acid or glutamic acid are suitable

### examples.

Preferably, the water-soluble compound consists of particles of a weight average size of greater than or equal to 0.001 mm and smaller than or equal to 3 mm, preferably of greater than or equal to 0.01 mm and smaller than or equal to 2 mm. The skilled person will recognize that also other combinations of those lower and upper limits to be practicable. The indicated size of the particles is measured by conventional sieve analysis and averaged according to weight. The measurement is taken prior to inclusion of the particles into the body care composition.

Particles of that size are big enough to remain partially non-dissolved and show an effect during the cleaning process and small enough to be dissolved in acceptable time during the rinsing process. Even if the particles are not completely dissolved during rinsing, it has been found, that their removal is improved. This is probably a consequence of the decreasing particle size and the reduction of adhesion by dissolving on the surface. The particles that are removed prior to their total dissolution will finally dissolve in the sink with the rest of the waste water, so that blocking of the pipes, due to deposition build-up is not possible.

In a preferred composition according to the present invention the particles remain suspended in the composition without separation or sedimentation over time. This can be achieved in any conventional manner, such as adjusting all components to have comparable density parameters, or by use of materials which adjust the rheological behaviour such that the particles remain suspended. Such materials can provide a thixotropic behaviour or create network where the particles remain suspended below the flow limit also referred to as liquid yield point of the composition.

In a further embodiment, the body care composition according to the present invention comprises at least two water-soluble compounds, which have a water-solubility at 20 °C and neutral pH of greater than or equal to 0.1 % and smaller than or equal to 7 %, preferably greater than or equal to 0.5% and smaller than or equal to 5 %, and are at least partially non-dissolved in the body care composition and have different average particle sizes.

This is advantageous, when stepwise removal behaviour is desired. For example, particles with a greater size but a better solubility can be used for a rough cleaning, whereas for a finer cleaning smaller particles of a material that is less soluble are suitable.

In addition to the soluble solids the body care composition can also comprise at least one not water-soluble compound, consisting of particles of the average size of greater than or equal to 0.001 mm and smaller than or equal to 3 mm, preferably of greater than or equal to 0.01 mm and smaller than or equal to 2 mm. The skilled person will consider also other combinations of those lower and upper limits to be practicable.

Further to the abrasive compounds, the body care composition can comprise further components, preferably selected from the group consisting of surfactants, emulgators, thickeners, acids, salts, bases, buffers, polymers, bleaching agents, colouring agents, optical brighteners, fragrances, preservatives, fillers, adjuvants, opacifiers, pearlescents, vitamins and/or compounds for the enhancement of the skin compatibility.

A particular object of the present invention is a cosmetic cleaning material, in particular a (peeling-) shampoo, (peeling-) shower bath, peeling-creme, peeling-mask, and paste for cleaning hands and/or intensive soap for rough staining.

Preferably, the body care composition is removed by rinsing after the cleaning process. During this removal the at least one water-soluble compound is dissolved fully or partially.

Examples 1 to 10 for compositions suitable as body care compositions according to the present invention are given below.

**Example 1 (Peeling-Shampoo):**

| | **wt.-%** |
|---|---|
| | |
| Dulcitol, grained | 15.00 |
| Sodium lauryl ether sulfate | 12.50 |
| Xanthan gum | 1.00 |
| Sodium formiate | 0.15 |
| Sodium benzoate | 0.35 |
| Sodium hydroxide | 0.22 |
| PEG-35 CASTOR OIL | 0.10 |
| Citric acid | 0.33 |
| Scent | 0.40 |
| Colouring agent | 0.05 |
| Sodium chloride | 1.00 |
| Water | add to 100 |

**Example 2 (Peeling-Shampoo):**

| | **wt**.-**%** |
|---|---|
| | |
| Asparagine, rough-crystalline | 15.00 |
| Acrylate Copolymer (30%) | 8.00 |
| Sodium lauryl ether sulfate | 40.00 |
| Sodium hydroxide | 1.50 |
| Cocoamidopropyl Betaine | 6.70 |
| Polyquaternium 39 (10%) | 2.10 |
| Tetrasodium EDTA | 0.05 |
| Preservative | 0.50 |
| Water | add to 100 |

**Example 3 (Shower Liquid):**

| | **wt.-%** |
|---|---|
| | |
| Asparic acid, rough-crystalline | 9.0 |
| Potassium hydrogentartrate, rough-crystalline | 6.0 |
| Sodium lauryl ether sulfate | 9.0 |
| Xanthan gum | 0.8 |
| Cocoamidopropyl Betaine | 4.0 |
| Disodium cocoglutamate | 0.5 |
| PEG-7 Glyceryl cocoate | 3.0 |
| Ethyleneglycol distearate | 1.0 |
| PEG-40 hydrated ricinus oil | 0.6 |
| Preservative | 0.5 |
| Sodium chloride | 1.0 |
| Colouring agent | 0.1 |
| Scent oil | 0.2 |
| Water | add to 100 |

**Example 4 (Showering Oil):**

| | **wt.-%** |
|---|---|
| | |
| Dulcitol, grained | 15.0 |
| Sun flower oil | add to 100 |
| Ricinus oil | 11.0 |
| Lauryl ether sulfate | 20.0 |
| Laureth-4 | 12.0 |
| Cocamide DEA | 8.0 |
| Scent oil | 1.5 |
| Preservative | 0.5 |

**Example 5 (Face Cleaning Lotion):**

| | **wt.-%** |
|---|---|
| | |
| Glutamic acid, finely-grained | 15.0 |
| C12-C14 fatty alcohol sulphosuccinate | 6.0 |
| lauryl polyglucoside | 4.0 |
| PEG-7 glyceryl cocoate | 3.0 |
| PEG-1200 glucose dioleate | 1.0 |
| Scent oil | 0.3 |
| Preservative | 0.7 |
| Water | add to 100 |

**Example 6 (Hand Washing Lotion):**

| | **wt.-%** |
|---|---|
| | |
| Saccharine, crystalline | 10.0 |
| Myristyl ether sulfate | 10.0 |
| Cocoamidopropyl Betaine | 2.0 |
| Laureth-2 | 2.0 |
| Ethyleneglycol distearate | 1.0 |
| Polyquaternium-10 | 0.5 |
| Scent oil | 0.5 |
| Sodium chloride | 1.0 |
| Colouring agent | 0.3 |
| Preservative | 0.2 |
| Water | add to 100 |

**Example 7 (Scrubbing Creme for the Removal of Horny Skin on Feet):**

| | **wt.-%** |
|---|---|
| | |
| Potassium hydrogentartrate, roughly-grained | 10.0 |
| Hard paraffine wax | 23.0 |
| Beeswax | 2.0 |
| Stearic acid | 2.0 |
| Cetyl stearate | 2.0 |
| Glycerine monostearate | 2.0 |
| Tris(hydroxymethyl)aminomethane | 0.2 |
| Scent oil | 0.2 |
| Preservative | 0.5 |
| Water | add to 100 |

**Example 8 (Hand Washing Gel):**

| | **wt.-%** |
|---|---|
| Zinc lactate | 15.0 |
| Sodium laurethyl sulfate | 10.0 |
| Disodium lauryl ether sulfosuccinate | 5.0 |
| PEG-120 methyl glucose dioleate | 1.0 |
| Hydroxyethyl cellulose | 1.0 |
| PPG-10 methyl glucose ether | 1.5 |
| Lauryl methyl gluceth-10 hydroxypropyl dimonium chloride | 3.0 |
| Scent oil | 0.2 |
| Preservative | 0.5 |
| Water | add to 100 |

**Example 9 (Make-Up Remover):**

| | **wt.-%** |
|---|---|
| | |
| Caffeine, finley-crystalline | 7.00 |
| Sodium borate | 0.60 |
| DMD hydantoine | 0.25 |
| Caprylic/Capryl triglyceride | 40.00 |
| Paraffinum liquidum | 2.00 |
| Beeswax | 10.00 |
| Lanolin | 1.00 |
| Polyglyceryl-10 decaoleate | 2.50 |
| Scent | 0.20 |
| Water | add to 100 |

**Example 10 (Liquid Soap):**

| | **wt.-%** |
|---|---|
| | |
| Lithium carbonate, medium-grained | 15.0 |
| Sodium lauryl ether sulfate | 20.9 |
| Hydrolyzed milk proteine | 3.0 |
| Preservatives | 0.3 |
| Water | add to 100 |

## Claims

1. A body care composition, comprising at least one water-soluble compound which has a water-solubility at 20 °C of greater than or equal to 0.1% and smaller than or equal to 7 % and is at least partially non-dissolved present in the material.

2. The body care composition according to claim 1, which comprises said at least one water-soluble compound in an amount greater than or equal to 0.5% and smaller than or equal to 70%, preferably greater than or equal to 5% and smaller than or equal to 60%.

3. The body care composition according to any of the preceding claims, wherein said at least one water-soluble compound has a water-solubility at 20 °C and neutral pH of greater than or equal to 0.2 % and smaller than or equal to 5 %, preferably of greater than or equal to 0.3 % and smaller than or equal to 4.5 %, more preferably of greater than or equal to 0.4 % and smaller than or equal to 4.2 % and most preferably of greater than or equal to 0.5 % and smaller than or equal to 4 %.

4. The body care composition according to any of the preceding claims, wherein said at least one water-soluble compound is selected from the group consisting of organic acids, inorganic acids, inorganic salts, organic salts, carbon hydrate derivatives, polyols, amino acids, acid amides, acid imides, salts of sulphonic acids and/or heterocycles.

5. The body care composition according to any of the preceding claims, wherein said at least one water-soluble compound is selected from the group consisting of adipic acid, azelaic acid, benzoic acid, sorbic acid, succinic acid, fumaric acid, mucic acid, phthalic acid, sulphanilic acid, *p*-aminobenzoic acid, hippuric acid, orotic acid, boric acid, magnesium lactate, calcium lactate pentahydrate, calcium glycerophosphate, tricalcium citrate, zinc benzoate, zinc lactate, potassium hydrogentartrate, lithium carbonate, potassium perchlorate, dulcitol, calcium gluconate, pentaerythrol, glutamine, glutamic acid, asparagine, aspartic acid, histidine, leucine, isoleucine, phenylalanine, tryptophane, methionine, saccharine, caffeine, theophylline and maltol.

6. The body care composition according to any of the preceding claims, wherein the water-solubility of the water-soluble compound depends on the pH, and wherein the maximum water-solubility of the water-soluble compound is achieved at a pH value of greater than or equal to 6.5 and smaller than or equal to 9.5.

7. The body care composition according to any of the preceding claims, wherein the non-dissolved part of the water-soluble compound consists of particles of a weight average size of greater than or equal to 0.001 mm and smaller than or equal to 3 mm, preferably of greater than or equal to 0.01 mm and smaller than or equal to 2 mm.

8. The body care composition according to any of the preceding claims, wherein the body care composition comprises two of said water-soluble compounds, which have different weight average particle sizes.

9. The body care composition according to any of the preceding claims, wherein the body care composition comprises additionally further components selected from the group consisting of surfactants, emulgators, thickeners, acids, salts, bases, buffers, polymers, bleaching agents, colouring agents, optical brighteners, fragrances, preservatives, fillers, adjuvants, opacifiers, pearlescents, vitamins and/or compounds for the enhancement of skin compatibility.

10. The body care composition according to any of the preceding claims, wherein the body care composition is a cosmetic cleaning material, in particular a peeling-shampoo or peeling-showerbath.
